# EUROPÄISCHE PATENTSCHRIFT

(11) **EP 4 103 693 B1**
(45) Veröffentlichungstag und Bekanntmachung des Hinweises auf die Patenterteilung: **03.12.2025**
(21) Anmeldenummer: 20792395.4
(22) Anmeldetag: 14.10.2020
(51) Int. Cl.: C12N 5/073, C12N 5/077, A23L 13/00

(54) **FLEISCHERZEUGUNG IN VITRO**
IN VITRO MEAT PRODUCTION
PRODUCTION IN VITRO DE VIANDE

(30) Priorität: 11.02.2020 DE 102020201661
(43) Veröffentlichungstag der Anmeldung: 21.12.2022
(73) Patentinhaber: Forschungsinstitut für Nutztierbiologie, 18196 Dummerstorf (DE)
(72) Erfinder: RÖNTGEN, Monika, 18147 Rostock (DE)
(74) Vertreter: Altmann Stößel Dick Patentanwälte PartG mbB
(86) Internationale Anmeldenummer: PCT/EP2020/078935
(87) Internationale Veröffentlichungsnummer: WO 2021/160301

(56) Entgegenhaltungen:
- WO-A2-2006/041429
- US-A1- 2012 213 743
- STANGE KATJA ET AL: "Methionine Sources Differently Affect Production of Reactive Oxygen Species, Mitochondrial Bioenergetics, and Growth of Murine and Quail Myoblasts In Vitro", INSTITUTE OF MUSCLE BIOLOGY AND GROWTH, RESEARCH INSTITUTE FOR FARM ANIMAL BIOLOGY (FBN), WILHELM-STAHL-ALLEE 2, 18196 DUMMERSTORF, GERMANY, vol. 45, no. 4, 23 March 2023 (2023-03-23), pages 2661 - 2680, XP93099502, DOI: 10.3390/cimb45040174
- MARIA CONCONI ET AL: "CD105(+) cells from Wharton's jelly show in vitro and in vivo myogenic differentiative potential", INTERNATIONAL JOURNAL OF MOLECULAR MEDICINE, 1 December 2006 (2006-12-01), Greece, pages 1089 - 1096, XP055757125, Retrieved from the Internet <URL:https://www.spandidos-publications.com/10.3892/ijmm.18.6.1089> DOI: 10.3892/ijmm.18.6.1089
- ÇETIN KOCAEFE ET AL: "Reprogramming of Human Umbilical Cord Stromal Mesenchymal Stem Cells for Myogenic Differentiation and Muscle Repair", STEM CELL REVIEWS, vol. 6, no. 4, 28 July 2010 (2010-07-28), US, pages 512 - 522, XP055757146, ISSN: 1550-8943, DOI: 10.1007/s12015-010-9177-7
- MAGALI KITZMANN ET AL: "The Muscle Regulatory Factors MyoD and Myf-5 Undergo Distinct Cell Cycle-specific Expression in Muscle Cells", THE JOURNAL OF CELL BIOLOGY, vol. 142, no. 6, 21 September 1998 (1998-09-21), US, pages 1447 - 1459, XP055756572, ISSN: 0021-9525, DOI: 10.1083/jcb.142.6.1447
- MARY LATIMER ET AL: "miR-210 expression is associated with methionine-induced differentiation of trout satellite cells", JOURNAL OF EXPERIMENTAL BIOLOGY, vol. 220, no. 16, 2 June 2017 (2017-06-02), pages 2932 - 2938, XP055756553, ISSN: 0022-0949, DOI: 10.1242/jeb.154484
- MRUNALINI K. GAYDHANE ET AL: "Cultured meat: state of the art and future", BIOMANUFACTURING REVIEWS, vol. 3, no. 1, 19 March 2018 (2018-03-19), XP055664794, ISSN: 2363-507X, DOI: 10.1007/s40898-018-0005-1
- THORREZ LIEVEN ET AL: "Challenges in the quest for 'clean meat'", NATURE BIOTECHNOLOGY, GALE GROUP INC., NEW YORK, US, vol. 37, no. 3, 1 March 2019 (2019-03-01), pages 215 - 216, XP036900611, ISSN: 1087-0156, [retrieved on 20190304], DOI: 10.1038/S41587-019-0043-0
- KOLKMANN A M ET AL: "Serum-free media for the growth of primary bovine myoblasts", CYTOTECHNOLOGY, KLUWER ACADEMIC PUBLISHERS, DORDRECHT, NL, vol. 72, no. 1, 28 December 2019 (2019-12-28), pages 111 - 120, XP037009076, ISSN: 0920-9069, [retrieved on 20191228], DOI: 10.1007/S10616-019-00361-Y
- MARKERT C D ET AL: "Mesenchymal Stem Cells: Emerging Therapy for Duchenne Muscular Dystrophy", PM&R, ELSEVIER, AMSTERDAM, NL, vol. 1, no. 6, 1 June 2009 (2009-06-01), pages 547 - 559, XP026459361, ISSN: 1934-1482, [retrieved on 20090611]
- SWIERCZEK BARBARA ET AL: "From pluripotency to myogenesis: a multistep process in the dish", JOURNAL OF MUSCLE RESEARCH AND CELL MOTILITY, CHAPMAN, LONDON, GB, vol. 36, no. 6, 29 December 2015 (2015-12-29), pages 363 - 375, XP035922000, ISSN: 0142-4319, [retrieved on 20151229], DOI: 10.1007/S10974-015-9436-Y
- KUANG ET AL: "The emerging biology of satellite cells and their therapeutic potential", TRENDS IN MOLECULAR MEDICINE, ELSEVIER CURRENT TRENDS, GB, vol. 14, no. 2, 22 January 2008 (2008-01-22), pages 82 - 91, XP022477482, ISSN: 1471-4914, DOI: 10.1016/J.MOLMED.2007.12.004

## Beschreibung

Die vorliegende Erfindung bezieht sich auf eine Methode zur Erzeugung einer Zusammensetzung, welche tierisches Protein enthält Die vorliegende Erfindung bezieht sich außerdem auf die Verwendung eines Nährmediums, welches einen im Vergleich zum Standardmedium reduzierten Gehalt an Methionin von höchstens 5 µM aufweist, zur Differenzierung von mesenchymalen Stammzellen aus einer Nabelschnur eines landwirtschaftlichen Nutztiers und auf eine Methode zur *in vitro* Herstellung einer fleischähnlichen Zusammensetzung.

Bis 2050 wird sich die Weltbevölkerung auf etwa 10 Milliarden erhöhen. Eine der wichtigsten Herausforderungen in diesem Zusammenhang ist die Sicherstellung der Ernährung im globalen Maßstab und speziell die Versorgung mit hochwertigem Protein. Dabei spielt die konventionelle Produktion von Fleisch mit landwirtschaftlichen Nutztieren eine wichtige Rolle; sie hat sich in den letzten 50 Jahren bereits verdreifacht. Da trotz des steigenden Bedarfes negative Effekte auf die Umwelt und das Klima vermieden werden sollen und eine nachhaltige Fleischproduktion die Reduktion der Tierzahlen voraussetzt, müssen alternative Produktionsformen gefunden werden. Eine Option ist sogenanntes "In-vitro-Fleisch", welches als umwelt- bzw. ressourcenschonende Alternative zu Fleisch angesehen wird (Stephens et al., 2018; Thorrez & Vandenburgh, 2019). Gaydhane et al. diskutieren die Vor- und Nachteile von kultiviertem Fleisch oder In-vitro-Fleisch (Mrunalini K. Gaydhane, Urbashi Mahanta, ·Chandra S. Sharma, Mudrika Khandelwal, Seeram Ramakrishna, Biomanufacturing Reviews, Bd. 3, Nr. 1, 19. März 2018).

Obwohl die grundlegende Methode zur Herstellung von Fleisch aus tierischen Zellen (Rind, Huhn, Seafood) bekannt ist, wird es bisher nur im Labormaßstab, d.h. in geringen Mengen hergestellt und ist daher noch sehr kostenintensiv. Bisher wird zur Gewinnung der Ursprungszellen meist Muskelgewebe von Schlachttieren verwendet oder es werden Muskelbiopsien an lebenden Tieren durchgeführt, so dass weiterhin ethische Probleme bestehen. Bei den gewonnenen Zellen handelt es sich um adulte Stammzellen, sogenannte Satellitenzellen, die in ihrer Proliferations- und Differenzierungsfähigkeit bereits deutlich eingeschränkt sind. Sie weisen außerdem einen hohen Grad an Heterogenität auf und die Zellqualität unterliegt in Abhängigkeit vom Spendertier starken Schwankungen, die z.B. durch Unterschiede in Alter und Genetik bedingt sind und aus Umwelteinwirkungen resultieren.

Alternativ verwendet die Firma "Meatable" überwiegend hämatopoetische Stammzellen vom Rind, die aus Nabelschnurblut gewonnen werden. Hämatopoetische Stammzellen müssen allerdings genetisch manipuliert (reprogrammiert) werden, um Muskel- und Fettzellen bilden zu können.

WO 2006/041429 A1 beschreibt den Einsatz von embryonalen Stammzellen aus befruchteten Eizellen zur Herstellung von Fleischprodukten. Die Isolierung von Zellen aus Nabelschnurgewebe wurde für verschiedene Spezies, am häufigsten für den Menschen (Ishige et al., 2009; Corotchi et al., 2013), aber auch für Wiederkäuer (z.B. Cardoso et al., 2012) und Pferd (Hoynowski et al., 2007) beschrieben. Die Isolierung mesenchymaler Stammzellen (MSC) aus der Nabelschnurmatrix des Schweines wurde von Carlin et al. beschrieben (Carlin et al., 2006).

Grundsätzlich können mesenchymale Stammzellen (MSC) durch Auswachsen der Zellen aus Gewebeexplantaten oder durch enzymatischen Verdau des Gewebes gewonnen werden (Moretti et al., 2010). Für den enzymatischen Verdau werden u.a. Collagenasen, Trypsin, Dispase, und Hyaluronidase genutzt die aus tierischen Materialien stammen und damit ein Kontaminationsrisiko mit sich bringen (Marcus-Sekura et al., 2011). Weiterhin wurde nachgewiesen, dass die Effizienz der Isolierung (Ausbeute an vitalen Zellen) bei Nutzung der Explant-Methode besser ist, da Zellschädigungen vermieden werden (z.B. Arutyunyan et al., 2016).

Da MSC aus Nabelschnurgewebe (UC-MSC) bereits in einer frühen Phase der Embryogenese in die Nabelschnur einwandern, zeichnen sie sich durch eine einzigartige Mischung der Eigenschaften pränataler (embryonaler) und postnataler (adulter) Stammzellen aus (Wang et al., 2004; Arutyunyan et al., 2016). UC-MSC haben zum Beispiel eine hohe Biosicherheit, da sie kein entzündliches oder tumorigenes Potential haben. Ein weiterer Vorteil von UC-MSC ist ihre hohe und langdauernde *in vitro*-Proliferationsrate (kurze Verdopplungszeit: 21 - 45 h, 7 bis etwa 25 serielle Passagen mit >300-facher Erhöhung der Zellzahl) bei stabilem Stammzell-Phänotyp, denn für die Herstellung von Zell-basiertem Fleisch ist die Produktion großer, homogener Zellmengen unter Beibehaltung der Stammzelleigenschaften (Moretti et al., 2010; Pham et al., 2016; Arutyunyan et al., 2016) eine entscheidende Voraussetzung. Die in dieser Hinsicht positiven Eigenschaften von UC-MSC resultieren unter anderem aus der Tatsache, dass sie noch Markerproteine embryonaler SC (z.B. Okt4, SOX2, Nanog) exprimieren (Beeravolu et al., 2016; Pham et al., 2016), welche die Differenzierung hemmen. Unter differenzierenden Bedingungen können sie außerdem eine Vielzahl von Zelltypen, wie Fettzellen, Muskelzellen, Knorpelzellen, Knochenzellen, Knochenmarksstroma-Zellen, aber auch Nervenzellen, Gefäßwandzellen, Herzmuskelzellen, Leber-ähnliche Zellen und β-Zellen des Pankreas, bilden (Ishige et al., 2009; Wang et al., 2011; Corotchi, 2013; Pham et al., 2016; Shivakumar et al., 2016). Positiv ist auch zu sehen, dass UC-MSC von Neugeborenen stammen und daher noch nicht durch Alterung und Umwelteinflüsse verändert wurden.

Die Umwandlung der UC-MSC in myogene Zellen bzw. die Bildung von Muskelfasern ist das Kernstück auf dem Weg zu Herstellung von Zell-basiertem Fleisch. Die Differenzierung von UC-MSC in Muskelzellen wurde bisher jedoch nur wenig untersucht. Als Möglichkeit, myogene Differenzierung zu induzieren wurde Kultivierung in myogenem Medium unter Serumentzug und Zugabe von 5-Azacytidin (Conconi et al., 2006) beschrieben; alternativ wurden Zellen mit myogenen Genen transfiziert (MyoD: Kocaefe et al., 2010). Die Differenzierung von MSC in Muskelzellen wurde also bisher chemisch oder durch genetische Manipulation durchgeführt. Bei adulten Muskelstammzellen wird die Induktion durch den Entzug oder die Reduktion von fetalem bovinen Kälberserum (FBS), einem wesentlichen Bestandteil von Zellkulturmedien, erreicht.

Trotz des bestehenden Wissens besteht ein hoher Bedarf an verbesserten Mitteln und Methoden zur in vitro Herstellung von fleischähnlichen Erzeugnissen, speziell in großem Maßstab. Dieses Problem wird durch die Gegenstände der unabhängigen Ansprüche gelöst, optional ergänzt durch die Merkmale der abhängigen Ansprüche und der vorliegenden Beschreibung.

Deshalb bezieht sich die vorliegende Erfindung auf eine Methode zur Erzeugung einer Zusammensetzung, welche tierisches Protein enthält, umfassend
(a) Isolierung von mesenchymalen Stammzellen aus einer Nabelschnur eines landwirtschaftlichen Nutztieres, bevorzugt aus der Familie Suidae;
(b) Inkubieren der mesenchymalen Stammzellen unter Bedingungen, die zu einer myogenen Differenzierung der mesenchymalen Stammzellen führen; und
(c) Ernten der myogen differenzierten Zellen.

Im Rahmen der vorliegenden Beschreibung werden die Begriffe "beinhalten", "umfassen", oder "einschließen" und jegliche grammatikalischen Variationen davon in nichtausschließlicher Bedeutung verwendet. Diese Begriffe können sich also einerseits auf eine Situation beziehen, in der neben den Merkmalen, die durch die Begriffe eingeführt werden, keine weiteren Merkmale in dem beschriebenen Gegenstand vorhanden sind, aber auch auf eine Situation, in der eines oder mehrere weitere Merkmale vorhanden sind. So können zum Beispiel die Ausdrücke "A umfasst B", "A enthält B" und "A schließt B ein" sowohl auf eine Situation hindeuten, in der neben B kein weiteres Element in A vorhanden ist (also eine Situation, in der A ausschließlich aus B besteht), aber auch auf eine Situation in der, neben B ein oder mehrere weitere Elemente in A vorhanden sind, wie etwa Element C, Elemente C und D oder sogar weitere Elemente. Entsprechend beziehen sich die Ausdrücke "umfassend ein" und "enthaltend ein" bevorzugt auf "umfassend ein oder mehrere"; d.h. sind äquivalent zu "umfassend mindestens ein".

Weiterhin werden im Folgenden die Begriffe "bevorzugt", "mehr bevorzugt", "noch mehr bevorzugt", "am meisten bevorzugt", "im Besonderen", "spezifisch" oder ähnliche Begriffe im Zusammenhang mit optionalen Merkmalen verwendet, ohne weitere Möglichkeiten auszuschließen. Merkmale, die durch diese Begriffe eingeführt werden, sind also als optionale Merkmale zu verstehen und beschränken den Umfang der Ansprüche in keiner Weise. Wie der Fachmann verstehen wird, kann die Erfindung unter Verwendung alternativer Merkmale durchgeführt werden. In gleicher Weise sind Merkmale, die durch einen Ausdruck wie "in einer Ausführungsform" oder ähnlichen Formulierungen eingeführt werden, als optionale Merkmale zu verstehen, ohne die Möglichkeit der Ausgestaltung weiterer Ausführungsformen zu beschränken, ohne die Erfindung zu beschränken und ohne die Möglichkeit, weitere Kombinationen von Merkmalen mit anderen optionalen oder nicht optionalen Merkmalen zur Verfügung zu stellen, einzuengen

Der Begriff "Standardbedingungen" bezieht sich, soweit nicht anders angegeben, auf IUPAC Standard Umgebungs-Temperatur und -Druckbedingungen (Standard Ambient Temperature and Pressure, SATP), d.h. bevorzugt auf eine Temperatur von 25 °C und einen absoluten Druck von 100 Kilopascal; ebenfalls bevorzugt umfassen Standardbedingungen zusätzlich einen pH von 7. Sofern nicht anders angegeben, bezieht sich der Begriff "ungefähr" auf den angegebenen Wert mit der allgemein akzeptierten technischen Genauigkeit im relevanten Fachgebiet, bevorzugt bezieht sich der Ausdruck auf den angegebenen Wert ± 20%, bevorzugt ± 10%, mehr bevorzugt ± 5%. Weiterhin bezieht sich der Begriff "im Wesentlichen" darauf, dass solche Abweichungen nicht gegeben sind, die einen Einfluss auf das angegebene Ergebnis haben; das heißt, mögliche Störungen führen zu einer Abweichung von nicht mehr als ± 20%, bevorzugt ± 10%, mehr bevorzugt ± 5% vom angegebenen Wert. Der Begriff "im Wesentlichen bestehend aus" bezieht sich deshalb auf die angegebenen Komponenten unter Ausschluss anderer Komponenten, außer solchen Komponenten, die als Verunreinigungen vorhanden sind, von Komponenten, die als nicht vermeidbare Komponenten Ergebnis des Herstellungsprozesses sind und von Komponenten, die zu einem von dem technischen Effekt der Erfindung verschiedenen Zweck zugefügt wurden. Zum Beispiel kann eine Zusammensetzung, die als "bestehend im Wesentlichen aus" beschrieben wird, zusätzlich übliche Additive, Excipienten, Verdünnungsmittel, Trägerstoffe oder ähnliches enthalten. Bevorzugt enthält eine Zusammensetzung, die im Wesentlichen aus einem Satz von Komponenten besteht, weniger als 5% (w/w), bevorzugt weniger als 3% (w/w), mehr bevorzugt weniger als 1% (w/w), noch mehr bevorzugt weniger als 0.1% (w/w) von nicht spezifizierten Komponenten.

Die Methode zur Erzeugung einer Zusammensetzung, welche tierisches Protein enthält, ist ein *in* vitro-Verfahren. Die erfindungsgemäße Methode kann zusätzliche Schritte zu den ausdrücklich erwähnten enthalten; zum Beispiel können sich weitere Schritte auf das Bereitstellen von Nabelschnur beziehen oder auf weitere Inkubations- und/oder Vermehrungsschritte mit den erhaltenen myogen differenzierten Zellen, z.B. umfassend einen oder mehrere Schritte der Vermehrung der mesenchymalen Stammzellen und/oder der erhaltenen myogen differenzierten Zellen, und/oder umfassend einen Schritt der dreidimensionalen Anordnung der Zellen und bevorzugt weitere Vermehrung. Einer oder mehrere der genannten Schritte können auch in automatisierter Art und Weise durchgeführt werden.

Der Begriff "Tier" bezeichnet im Kontext der vorliegenden Erfindung einen heterotrophen Vertreter der Domäne Eukarya, welcher einen Organismus bildet, der Muskelzellen enthält. Der Begriff "landwirtschaftliches Nutztier" wird hierin also ausschließlich für landwirtschaftliche Nutztiere verwendet, die Säugetiere sind. Im Rahmen der vorliegenden Erfindung werden mesenchymalen Stammzellen aus einer Nabelschnur eines landwirtschaftliche Nutztiers, bevorzugt eines Mitglieds der Familie Suidae, noch mehr bevorzugt eines Vertreters der Gattung Sus, am meisten bevorzugt eines Vertreters der Art Sus scrofa, verwendet.

Der Begriff "tierisches Protein" wird hierin für jegliches Eiweiß tierischen Ursprungs verwendet. Bei dem tierischen Protein handelt es sich also um mindestens ein Polypeptid, dessen Aminosäuresequenz der Aminosäuresequenz eines Proteins mindestens eines Tieres entspricht, bevorzugt im Wesentlichen damit identisch ist. Der Begriff "Protein" wird hierbei in Übereinstimmung mit dem üblichen Gebrauch des Begriffes sowohl für eine einzelne Polypeptidspezies verwendet, als auch für eine Mischung mehrerer, bevorzugt vieler, verschiedener Polypeptidspezies. Das tierische Protein enthält also bevorzugt eine Mischung verschiedener Polypeptidspezies. Bevorzugt ist das tierische Protein in einer tierischen Zelle enthalten und/oder wurde von einer tierischen Zelle produziert.

Der Begriff "Zusammensetzung" wird hierin für jegliche Präparation verwendet, welche die angegebene(n) Komponente(n) enthält. Der Begriff "Zusammensetzung, welche tierisches Protein enthält" umfasst also alle Präparationen, die tierisches Protein enthalten, ungeachtet des Vorhandenseins und/oder des Anteils weiterer Komponenten, und ungeachtet des Protein- und/oder Wassergehaltes der Zusammensetzung. Bevorzugt enthält die Zusammensetzung mindestens 5% (w/w), mehr bevorzugt mindestens 10% (w/w), noch mehr bevorzugt mindestens 25% (w/w) Protein. Ebenfalls bevorzugt enthält die Zusammensetzung mindestens 1% (w/w) Lipide, mehr bevorzugt mindestens 5% (w/w) Lipide. Bevorzugt enthält die Zusammensetzung 1% (w/w) bis 25% (w/w) Lipide, mehr bevorzugt 5% (w/w) bis 15% (w/w) Lipide. Methoden zur Bestimmung des Eiweiss- und Fettgehalts sind dem Fachmann aus allgemeinen Lehrbüchern bekannt, z.B. AOAC International Official Methods for Analysis 17th Edition, AOAC International, Gaithersburg, MD, 2000; bevorzugt sind die Kjeldahl-Methode zur Proteinbestimmung (Simonne et al., (1997), Journal of the Science of Food and Agriculture 73(1):39-45) und die Methode nach Soxhlet zur Bestimmung des Fettgehalts (Bastian et al. (1985), J Assoc Off Anal Chem 68(5):876-880). Bevorzugt enthält die Zusammensetzung tierische Zellen, bevorzugt tierische Muskelzellen; mehr bevorzugt besteht die Zusammensetzung im Wesentlichen aus tierischen Zellen, bevorzugt aus tierischen Muskelzellen. Bevorzugt enthält die Zusammensetzung Muskelfasern, es handelt sich bei der Zusammensetzung also bevorzugt um eine fleischähnliche Zusammensetzung, bevorzugt mit fleischartiger Textur; entsprechend weist die Zusammensetzung bevorzugt einen Wassergehalt von 40% (w/w) bis 75% (w/w), bevorzugt von 50% (w/w) bis 65% (w/w), auf. Bei der Zusammensetzung kann es sich aber auch um eine getrocknete Zusammensetzung mit einem Wassergehalt unter 10% (w/w), bevorzugt unter 5% (w/w) handeln.

Mit dem Begriff "Vorläuferzellen" werden hierin Zellen bezeichnet, die die Fähigkeit aufweisen, zu Myocyten oder Myocyten-ähnlichen Zellen zu differenzieren. Bevorzugt produzieren die Vorläuferzellen kein Desmin, kein F-Aktin, kein MyoD, kein MyoG und/oder kein Myosin; entsprechend sind Vorläuferzellen bevorzugt keine Myocyten oder Myocyten-ähnlichen Zellen. Bevorzugt exprimieren die Vorläuferzellen mindestens einen Marker ausgewählt aus CD73, CD90, CD105, Oct4, Nanog, und Sox2. Im Rahmen der vorliegenden Erfindung werden mesenchymale Stammzellen verwendet, bevorzugt perinatale mesenchymale Stammzellen. Bevorzugt können die Vorläuferzellen mindestens in Myocyten und in Adipocyten differenzieren.

Der Begriff "perinatales Gewebe" wird hierin für alle Gewebe verwendet, die zumindest teilweise vom Fötus eines Säugetieres gebildet werden, aber nicht Teile des eigentlichen Fötus sind. Insbesondere bezeichnet der Begriff alle Gewebe bzw. Organe, die Teil der Nachgeburt eines Säugetieres sind, also insbesondere Nabelschnur, Plazenta, und/oder Eihaut. Im Rahmen der vorliegenden Erfindung wird die Nabelschnur eines landwirtschaftlichen Nutztiers oder ein Teil davon verwendet. Derartiges perinatales Gewebe wird bevorzugt nicht invasiv im Rahmen der Geburt gewonnen, bevorzugt nach Ausscheidung des perinatalen Gewebes aus dem mütterlichen Organismus.

Der Begriff "Vermehren von mesenchymalen Stammzellen " wird hierin in der üblichen, dem Fachmann geläufigen, Bedeutung benutzt. Der Begriff bezieht sich also bevorzugt auf die Inkubation von mesenchymalen Stammzellen unter Bedingungen, die eine Zellteilung zumindest eines Teils der mesenchymalen Stammzellen erlauben und so bevorzugt zu einer Zunahme der Gesamtzahl der mesenchymalen Stammzellen führen. Bevorzugt umfasst das Vermehren der mesenchymalen Stammzellen ein Einbringen der mesenchymalen Stammzellen in ein Nährmedium, bevorzugt in ein Nährmedium, welches frei von bovinem Serum ist, mehr bevorzugt in ein Nährmedium, welches frei von Serum eines landwirtschaftlichen Nutztieres ist, noch mehr bevorzugt in ein Nährmedium, welches frei von Serum eines Säugetieres ist, am meisten bevorzugt in ein serumfreies Nährmedium. Das Nährmedium enthält bevorzugt einen Anteil konditioniertes Nährmedium, bevorzugt eines der oben beschriebenen serumfreien Nährmedien. Bevorzugt beträgt der Anteil an konditioniertem Medium 5% (v/v) bis 50% (v/v), mehr bevorzugt 10% (v/v) bis 40% (v/v), noch mehr bevorzugt 15% (v/v) bis 30% (v/v), am meisten bevorzugt ungefähr 20% (v/v) am Gesamtvolumen des Mediums. Der Begriff "konditioniertes Medium" bezieht sich hierbei bevorzugt auf ein Medium, welches dadurch gewonnen wurde, dass Vorläuferzellen, bevorzugt der Passagen 1 oder 2 nach Isolierung, in dem Medium für mindestens einen, mehr bevorzugt mindestens zwei, noch mehr bevorzugt mindestens drei, Tag(e) inkubiert wurden. Ebenfalls bevorzugt kann das serumfreie Medium Komponenten enthalten, die aus dem vorbezeichneten konditionierten Medium erhalten wurden. Bevorzugte serumfreie Medien werden hierin in den Ausführungsbeispielen beschrieben.

Der Begriff "myogene Differenzierung" ist dem Fachmann grundsätzlich bekannt. Im Kontext der vorliegenden Beschreibung bezeichnet der Begriff die Entwicklung von mesenchymalen Stammzellen zu Myocyten oder myocyten-ähnlichen Zellen, in einer bevorzugten Ausführungsform zu Muskelfasern. Diese Entwicklung kann durch Bestimmung einer Abnahme der Expression von Markern von mesenchymalen Stammzellen, insbesondere der an anderer Stelle hierin beschriebenen, und/oder bevorzugt durch Bestimmung einer Zunahme der Expression von Myocyten-Markern, insbesondere Desmin, F-Actin, MyoD, MyoG und/oder Myosin verifiziert werden. Entsprechende Methoden sind dem Fachmann bekannt und werden hierin in den Ausführungsbeispielen gezeigt.

Bedingungen, die eine myogene Differenzierung von Vorläuferzellen induzieren, sind dem Fachmann grundsätzlich bekannt. So können mesenchymale Stammzellen genetisch oder chemisch so manipuliert werden, dass sie eine myogene Differenzierung durchlaufen, z.B. durch Überexpression des Faktors MyoD, Absenken des Serumgehalts des Nährmediums von 10 bis 20% auf meist 2%, und/oder durch Inkubation in einem Medium, welches 5-Azacytosin enthält. Erfindungsgemäß umfassen Bedingungen, die zu einer myogenen Differenzierung von mesenchymalen Stammzellen führen, eine Inkubation in einem, bevorzugt serumfreien, Nährmedium, welches einen im Vergleich zum Standardmedium (bevorzugt Dulbecco's modifiziertes Eagle Medium; DMEM: 30 mg/l Methionin, entsprechend 200 µM Methionin) reduzierten Gehalt an Methionin von höchstens 5 µM aufweist, bevorzugt höchstens 4 µM, mehr bevorzugt höchstens 3 µM, noch mehr bevorzugt höchstens 2,5 µM. Am meisten bevorzugt umfassen Bedingungen, die zu einer myogenen Differenzierung von mesenchymalen Stammzellen führen, Inkubation in einem Nährmedium ohne zugesetztes Methionin. In einer bevorzugten Ausführungsform umfassen Bedingungen, die zu einer myogenen Differenzierung von mesenchymalen Stammzellen führen, also eine Inkubation in einem, bevorzugt serumfreien, Nährmedium, welches kein Methionin enthält (d.h. eine Konzentration von 0 µM Methionin als freie Aminosäure).

Bevorzugt wird während der myogenen Differenzierung ein Kontakt der mesenchymalen Stammzellen mit einer festen Oberfläche vermieden; insbesondere wird bevorzugt ein Anheften der mesenchymalen Stammzellen an ein festes oder halbfestes Substrat vermieden. Ebenfalls bevorzugt wird während der Differenzierung eine Zusammenlagerung der Zellen gefördert, um die Ausbildung einer 3D-Struktur zu fördern. Bevorzugt werden die mesenchymalen Stammzellen deshalb in einem hängenden Tropfen inkubiert; entsprechende Methoden sind dem Fachmann bekannt.

In den der vorliegenden Erfindung zugrundeliegenden Versuchen wurde überraschend festgestellt, dass sich aus perinatalen Geweben, erfindungsgemäß aus Nabelschnur eines landwirtschaftlichen Nutztiers, mesenchymale Stammzellen isolieren lassen, die eine myogene Differenzierung durchlaufen können. Überraschenderweise wurde gefunden, dass eine solche Differenzierung vorteilhaft durch Inkubation in einem Medium mit einem reduziertem Methionin-Gehalt von höchstens 5 µM induziert werden kann. Überdies wurde überraschend festgestellt, dass sämtliche Schritte von der Isolierung von mesenchymalen Stammzellen über deren Vermehrung bis hin zur Bildung von myogen differenzierten Zellen in serumfreiem Medium durchgeführt werden können und keine genetische Manipulation der Zellen erfordern.

Die obigen Definitionen gelten mutatis mutandis für das Folgende. Zusätzliche Definitionen, die im Folgenden angewendet werden, gelten ebenfalls für alle hierin beschriebenen Ausführungsformen mutatis mutandis.

Schritt (a) der Methode der vorliegenden Erfindung umfasst bevorzugt:
(A) Bereitstellen von mindestens einem perinatalen Gewebe einer Nabelschnur, eines landwirtschaftlichen Nutztiers;
(B) zerkleinern des perinatalen Gewebes, so dass Stücke davon erhalten werden,
(C) Inkubieren der Stücke des perinatalen Gewebes in einem Nährmedium;
(D) wodurch mesenchymale Stammzellen erhalten werden.

Dies ist ein *in vitro*-Verfahren. Die Methode kann zusätzliche Schritte zu den ausdrücklich erwähnten enthalten; zum Beispiel können sich weitere Schritte auf eine Reinigung und/oder Desinfektion des perinatalen Gewebes vor Schritt B) beziehen. Einer oder mehrere der genannten Schritte können auch in automatisierter Art und Weise durchgeführt werden.

Der Schritt des Zerkleinerns des perinatalen Gewebes umfasst bevorzugt ein Zerschneiden des Gewebes. Bevorzugt werden hierbei Gewebestücke mit einem Volumen von 1 mm³ bis 500 mm³, bevorzugt 2 mm³ bis 100 mm³ produziert. Die Gewebestücke werden bevorzugt in eine Inkubationsvorrichtung mit mindestens einer festen Oberfläche eingebracht, bevorzugt ein Gefäß mit einer Plastikoberfläche, z.B. eine Petrischale. Bevorzugt werden Zellen und/oder Gewebe, die während der Inkubation an der genannten festen Oberfläche adhärieren, als Basis für die Isolierung von Vorläuferzellen verwendet. Das Medium, welches für die Inkubation des perinatalen Gewebes verwendet wird, ist bevorzugt serumfrei.

Die mit der erfindungsgemäßen Methode hergestellten myogen differenzierten Zellen sind verwendbar zur Herstellung von Muskel-, Fett- und/oder Bindegewebszellen.

Die Methode zur myogenen Differenzierung von mesenchymalen Stammzellen ist ein *in* vitro-Verfahren. Die Methode kann zusätzliche Schritte zu den ausdrücklich erwähnten enthalten; zum Beispiel können sich weitere Schritte auf eine Bereitstellung von V mesenchymalen Stammzellen und/oder eine Verwendung der erhaltenen myogen differenzierten Zellen beziehen. Einer oder mehrere der genannten Schritte können auch in automatisierter Art und Weise durchgeführt werden.

Die vorliegende Erfindung bezieht sich auch auf eine Verwendung eines Nährmediums, welches einen im Vergleich zum Standardmedium reduzierten Gehalt an Methionin von höchstens 5 µM aufweist, zur Differenzierung von mesenchymalen Stammzellen aus einer Nabelschnur eines landwirtschaftlichen Nutztiers. Wie oben dargestellt enthält das Nährmedium Methionin höchstens in einer Konzentration von 5 µM, bevorzugt höchstens 4 µM, mehr bevorzugt höchstens 3 µM, noch mehr bevorzugt höchstens 2,5 µM. Am meisten bevorzugt enthält das Nährmedium kein zugesetztes Methionin. Ebenfalls bevorzugt ist das Nährmedium ein serumfreies Nährmedium.

Die vorliegende Erfindung bezieht sich ebenfalls auf eine Methode zur Herstellung einer fleischähnlichen Zusammensetzung, umfassend
I) myogene Differenzierung von mesenchymalen Stammzellen gemäß der erfindungsgemäßen Methode zur myogenen Differenzierung von mesenchymalen Stammzellen;
II) dreidimensionale Anordnung der in Schritt I) erhaltenen differenzierten myogen differenzierten Zellen insbesondere durch Nutzung der Inkubation im "hängenden Tropfen" oder ähnlicher Verfahren, und
III) Vermehren der Zellen in der in Schritt II) erhaltenen dreidimensionalen Anordnung.

Die Methode zur Herstellung einer fleischähnlichen Zusammensetzung ist ein *in vitro -* Verfahren. Die Methode kann zusätzliche Schritte zu den ausdrücklich erwähnten enthalten; zum Beispiel können sich weitere Schritte auf das Bereitstellen von mesenchymalen Stammzellen, bevorzugt gemäß der erfindungsgemäßen Methode zur Herstellung von mesenchymalen Stammzellen, oder auf eine weitere Verarbeitung der fleischähnlichen Zusammensetzung beziehen. Einer oder mehrere der genannten Schritte können auch in automatisierter Art und Weise durchgeführt werden.

Der Begriff "fleischähnlichen Zusammensetzung" wird in breitem Sinn verwendet für Zusammensetzungen, die in Zusammensetzung, Textur und/oder Geschmack tierischem Fleisch ähneln und die für die Verwendung als Fleischersatz in mindestens einer Anwendung der Lebensmittelindustrie oder der Pharmazie verwendbar sind. Bevorzugte Anwendungen der Lebensmittelindustrie sind Wurstherstellung und Herstellung von Hackfleisch, insbesondere Burger-Patties. Bevorzugte Anwendung in der Pharmazie ist Muskelregeneration.

Der Begriff "dreidimensionale Anordnung" ist dem Fachmann geläufig. Bevorzugt werden myogen differenzierte Zellen und/oder daraus erzeugte Mikrogewebe im Raum angeordnet, bevorzugt eingebettet in eine feste oder bevorzugt halbfeste Matrix. Geeignete Matrixmaterialien sind dem Fachmann bekannt; bevorzugt sind biokompatible Matrixmaterialien, insbesondere proteinhaltige Matrices wie Gelatine und Fibrine, und polysaccharidhaltige Matrices wie Alginate, Chitosane, Hyaluronate und Agar-Agar. Die dreidimensionale Anordnung kann mit bekannten Verfahren erhalten werden, z.B. Einmischen von myogen differenzierten Zellen, bevorzugt Aggregaten davon, in verflüssigtes Matrixmaterial, Eingießen der Mischung in die gewünschte Form und anschließendes Verfestigen der Matrix. Alternativ oder zusätzlich können myogen differenzierte Zellen auch auf und/oder in eine feste Matrix auf- bzw. eingebracht werden, wobei die feste Matrix bevorzugt porös ist. Bevorzugt wird die dreidimensionale Anordnung durch 3D-Druck einer Mischung von myogen differenzierten Zellen oder myogen differenzierten Mikrogeweben und einem geeigneten Matrixmaterial hergestellt ("Bioprinting").

Bevorzugt werden in die dreidimensionale Anordnung zusätzlich adipogen differenzierte Zellen / Mikrogewebe eingeschlossen, wobei sich der Begriff "adipogen differenziert" auf Zellen bezieht, die Adipocyten oder adipocyten-ähnliche Zellen sind. Adipocyten und adipocyten-ähnliche Zellen lassen sich an der Bildung von intrazellulären Fetttröpfchen identifizieren, wie in den Ausführungsbeispielen dargestellt. Methoden zur adipogenen Differenzierung von Zellen sind dem Fachmann bekannt und werden in den Ausführungsbeispielen dargestellt. Bevorzugt werden die adipogen differenzierten Zellen aus den Vorläuferzellen der vorliegenden Erfindung hergestellt.

### Figuren

Fig. 1: Explant-Kultur mesenchymaler Stammzellen aus Nabelschnurgewebe vom Schwein: Explant-Kultur (A); typische Änderung der Morphologie über die Zeit (B).
Fig. 2: Expression von Markern durch UC-MSC, Nachweis über qRT-PCR; (A) CD73, (B) CD90, (C) CD105; als Negativkontrolle wurde Muskelzell-cDNA verwendet ("muscle cDNA").
Abb. 3: UC-MSC aus der Nabelschnur von neugeborenen Ferkeln differenzieren in Fett- (A) und Muskelzellen (B).
Abb. 4: Zeitverlauf der myogenen Differenzierung von C2C12-Mauszellen in verschiedenen Medien, Färbung mit einem Antikörper gegen Myosin (MF 20) an den Tagen 2 bis 5 nach Einbringen in das jeweilige Medium (d2-d5).
Abb. 5: Myogene Differenzierung von UC-MSC in Methionin-freiem Medium; (A) Durchlichtaufnahme myogen differenzierter Zellen; (B) spontane Bildung von Mikrogeweben.
Abb. 6: Mit der Methode des "hängenden Tropfens" hergestellte myogen differenzierte Mikrogewebe (Durchlicht-mikroskopische Aufnahme).

### Beispiel 1: Gewinnung von Nabelschnüren

Nabelschnüre von Schweinen wurden erst nach Beendigung des natürlichen Abnabelungsvorganges von der Mutter entnommen. Dazu wurden die Nabelschnüre nach Säuberung und Desinfektion mit 70%igem Äthanol etwa 2 cm vom Bauchnabel entfernt abgebunden bzw. abgeklemmt und mit einer scharfen Schere durchtrennt. Danach wurden die Nabelschnüre mehrfach mit steriler, eisgekühlter Dulbecco's Phosphat-gepufferter Kochsalzlösung mit 1% Penicillin und 1% Amphotericin B (DPBS-Spüllösung) gespült, bis Blutreste entfernt waren und das Gewebe weiß und sauber erschien. Anschließend wurden die Nabelschnüre für den Transport und bis zur weiteren Verwendung in frischer DPBS-Spüllösung auf Eis gelagert.

Explantkulturen wurden im Sterillabor angelegt. Frische Nabelschnüre wurden nach Überführung in den Steril-Bereich in 3 Schritten dekontaminiert und im weiteren Verlauf stets mit DPBS-Spüllösung feucht gehalten.

Dekontamination der Nabelschnur:
- Nabelschnur mit DPBS-Spüllösung mehrmals (3-5 mal) abspülen
- Nabelschnur in 70% EtOH tauchen (max. 1 min, um Gewebeschäden zu vermeiden)
- Nabelschnur mit DPBS-Spüllösung mehrmals (3-5 mal) abspülen

### Beispiel 2: Anlegen von Explantkulturen

Zum Anlegen der Explantkulturen wurden Stücke der Nabelschnur in eine kleine Petrischale mit DPBS-Spüllösung verbracht und danach longitudinal aufgeschnitten und aufgeklappt; das Gewebe wurde in ca. 4x4 mm große Stücke geschnitten bzw. mit einem Gewebeschneider ausgestanzt. Jeweils 20 Segmente wurden in eine 10 cm-CellCoat-Schale gegeben.

In jede Explantkultur (Kulturschale) wurden anschließend 10 ml Nährmedium gegeben und diese dann im Brutschrank bei 37°C, 5% CO₂ inkubiert. Varianten des Verfahrens (z.B. Entfernen der Nabelschnurgefäße, Isolieren von Wharton's Jelly) brachten keine signifikante Verbesserung des Ergebnisses.

### Beispiel 3: Prüfen des Effekts von Serum im Explantmedium

Explantversuche wurde mit Standardkulturmedium (DMEM low Glucose mit 10% FCS, 1% PenStrep, 1% Amphotericin) oder mit kommerziell erhältlichem Spezialmedium für mesenchymale Stammzellen (MesenCult ACF-Plus, 1% PenStrep, 1% Amphotericin, 2 mM L-Glutamin, 1x ACF Supplement (MC Stemcell Technologies, Köln; mit Attachment Faktor: #05448)) durchgeführt. MesenCult ACF-Plus enthält keine tierischen Zusätze oder Xenobiotika.

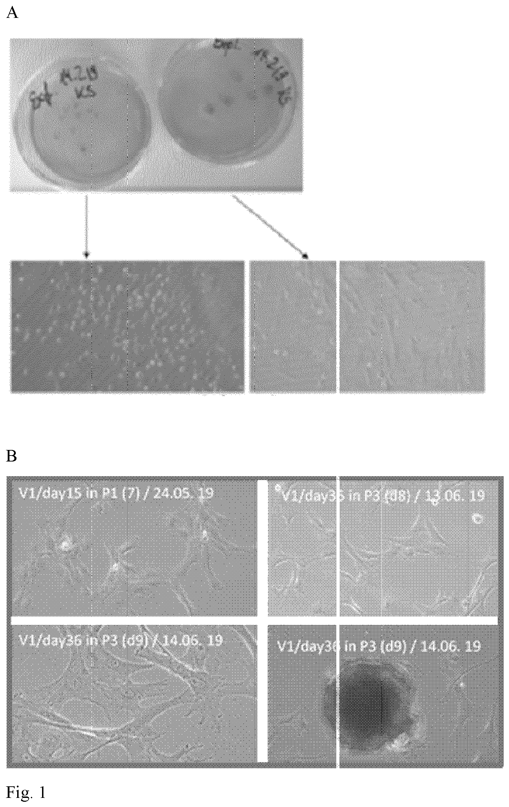

Mit allen Ansätzen und unabhängig von der Art des verwendeten Mediums waren nach 4-5 Tagen ausgewanderte Zellen (umbilical cord mesenchymal stem cells, UC-MSC) deutlich sichtbar vorhanden (Fig. 1); nach 6 bis 8 Tagen wurden die Gewebestücke entfernt und ein erster Mediumwechsel durchgeführt. Es konnte gezeigt werden, dass die Anwesenheit von FKS keinen Einfluss auf die Etablierung der Eltern-Zellbank (parent cell bank) nimmt.

### Beispiel 4: Bestimmung der Identität der UC-MSC

Die UC-MSC-Identität wurde durch das Vorhandensein verschiedener Oberflächenmarker nachgewiesen (Arutyunyan et al., 2016). Die Markerexpression von MSC ist heterogen, besonders charakteristisch sind jedoch CD73 (Wang et al., 2004; Corotchi, 2013), CD90 (Corotchi, 2013) und CD 105 (Wang et al., 2004; Corotchi, 2013), welche in ≥ 98% der Zellen vorhanden sein sollen.

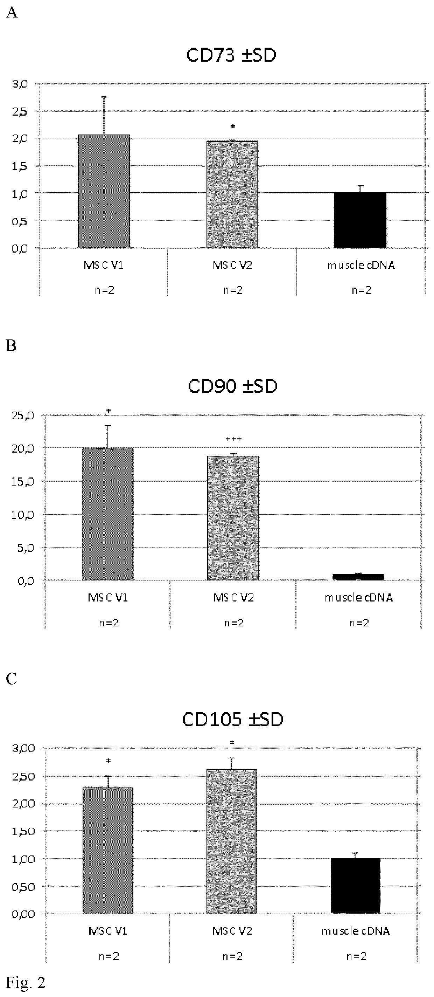

Für die Typisierung wurde zusätzlich qRT-PCR nach Standardmethoden eingesetzt. Die Ergebnisse für die Marker CD73, CD90 und CD105 sind in Fig. 2 gezeigt. Daneben werden die Pluripotenzgene Okt4, SOX2 und Nanog untersucht, ebenfalls mittels qR-PCR. Geeignete Primer sind in Tabelle 1 gezeigt.

### Beispiel 5: Tests auf Differenzierungsfähigkeit

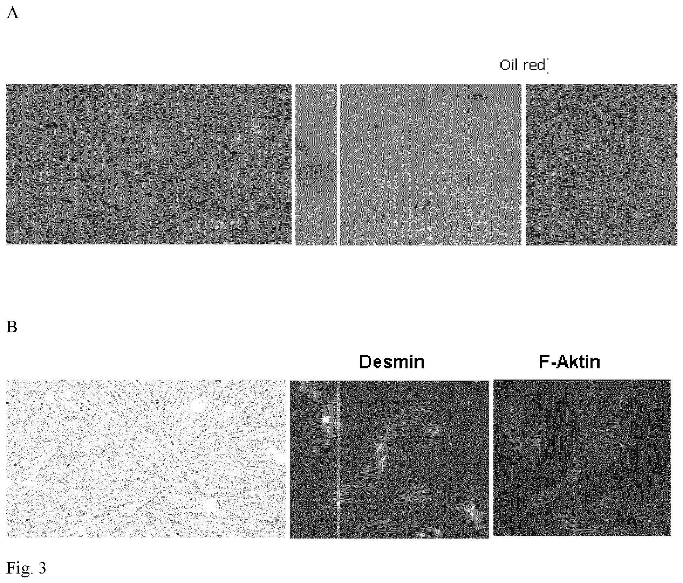

Um zu testen, ob sich die UC-MSC grundsätzlich in Muskel- und Fettzellen differenzieren lassen, wurden an Tag 6 der Kultur die erhaltenen Zellen enzymatisch abgelöst und in 6 Wells (2 Wells pro Kultur) einer 24-Well Primariaplatte ausgesät. Nach 13 Tagen in Kultur waren die Wells nahezu konfluent bewachsen und die Zellzahl hatte sich von 1,9 x 10⁵ auf 3,4 x 10⁵ erhöht. Um die Differenzierungsfähigkeit (Multipotenz) zu prüfen wurden ein Teil der Zellen in Kulturmedien verbracht, welche die Differenzierung in Fett- bzw. Muskelzellen fördern:
Die adipogene Differenzierung wurde durch Kultur in DMEM/F12 Medium mit 10% FBS, 2% Glutamin, supplementiert mit 1 mM Dexamethason, 500 µM IBMX (3-Isobutyl-1-methylxanthin), 100 µM Troglitazon und 1 µM/ml Insulin für die Dauer von 48 h induziert. Danach wurde das Induktionsmedium durch Differenzierungsmedium (DMEM/F12 Medium mit 10% FBS, 2% Glutamin, 1 µM/ml Insulin) ersetzt. Nach 3 Tagen waren erste Fetttröpfchen sichtbar und nach 5 Tagen wurden die Zellen fixiert und mit dem Farbstoff Oil red zum Fettnachweis gefärbt (Fig. 3A).

Die myogene Differenzierung wurde durch Kultur in DMEM mit 4,5 g/l Glucose (hohe Glukosekonzentration) mit 2% statt 10% FBS ausgelöst. Nach 4 Tagen wurden die Zellen fixiert und die Differenzierung mittels Fluoreszenz-markierten Antikörpern gegen die muskulären Proteine Desmin bzw. F-Aktin nachgewiesen (Fig. 3B).

Die Ergebnisse dieser Versuche zeigen, dass die isolierten Vorläuferzellen zu myogener und adipogener Differenzierung befähigt sind.

### Beispiel 6: Optimierung der myogenen Differenzierung

Die bekannten Methoden zur myogenen Differenzierung verwenden serumhaltige Kulturmedien und zeichnen sich durch eine häufig geringe Effizienz aus. Ziel war es deshalb, eine hohe Effizienz der Differenzierung bei ausschließlicher Nutzung natürlicher, physiologischer Prozesse, d.h. ohne chemische oder genetische Manipulation zu erreichen.

Für die Induktion der Differenzierung wurde Methionin-reduziertes bzw. -freies Medium genutzt, welches, ohne an theoretische Erwägungen gebunden sein zu wollen, wahrscheinlich über eine DNA-Demethylierung zur Expression von myogenen Genen (MyoD und MyoG) führt.

In den Differenzierungsversuchen wurden folgende Medien verwendet:
- "FCS": DMEM high Glucose, 2% FCS (fetales Kälberserum), 0.1% P/S (Penicillin/Streptomycin)
- "HS": DMEM high Glucose, 10% HS (Horse Serum), 0.1% P/S
- "HS+AZA": DMEM high Glucose, 5% HS, 0.1% P/S, 10 µM Azacytidin
- "-Met+Glu": DMEM high Glucose -MQC (DMEM high Glucose ohne Methionin, Glutamin, Cystin, Gibco, 21013-024), 5% HS, 0.1% P/S, 4 mM L-Glutamin
- "2,5 µM Met": DMEM high Glucose -MQC, 5% HS, 0.1% P/S, 2 mM L-Glutamin, 2,5 µM L-Methionin
- "5 µM Met": DMEM high Glucose -MQC, 5% HS, 0.1% P/S, 2 mM L-Glutamin, 5 µM L-Methionin

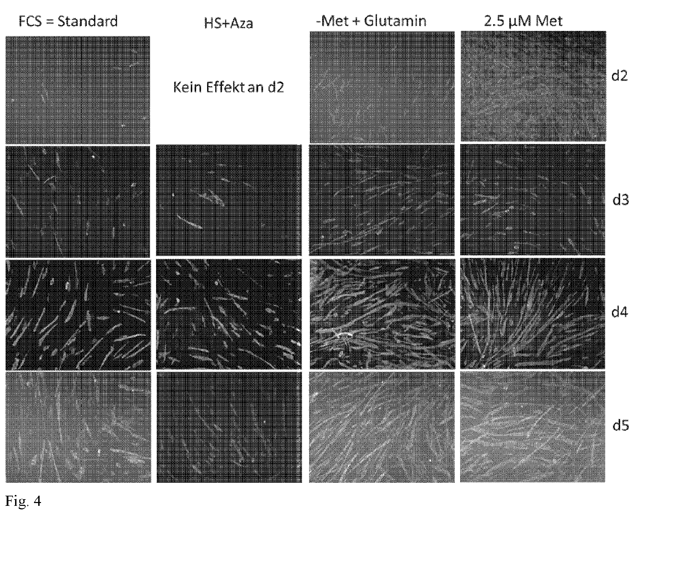

Die verwendete Zellinie war die Mauszelllinie C2C12. Die Ergebnisse sind in Fig.4 dargestellt, in der vergleichend das Standardmedium (mit reduzierter Serumkonzentration = FCS), die chemische Induktion (Medium mit 10 µM 5-Azacytidine = HS+AZA), und Medien ohne Methionin (aber supplementiert mit 4 mM Glutamin = -Met+Glutamin) bzw. mit reduzierter Methioninkonzentration (2,5 µM supplementiert mit 2 mM Glutamin = 2,5 µM Met) verglichen werden. Es wurde auch geprüft, ob es günstiger ist, das Differenzierungsmedium nach 48 Stunden abzusetzen. Da kein optimierender Effekt eintrat, wurde das Differenzierungsmedium für 5 Tage im Ansatz belassen.

Zum Nachweis der myogenen Differenzierung wurden Antikörper gegen das Muskelprotein Desmin (mouse anti-Desmin, clone D-33, DAKO) und ein Antikörper gegen Myosin (MF 20, anti-sarcomere (MHC), Eigenproduktion mit Hybridomakulturen zur Verfügung gestellt von Dr. Julia v. Maltzahn, Leibniz-Institut für Alternsforschung, Jena) genutzt. Fig.4 zeigt Färbungen mit MF 20, da die Expression von sarkomerischem Myosin besonders relevant für den Differenzierungserfolg ist.

Der Zeitverlauf der verschiedenen Ansätze (Fig. 4) zeigt deutlich, dass das von uns entwickelte Medium sogar einen größeren Differenzierungserfolg aufweist als das Standardmedium. Die Differenzierung beginnt früher, die Muskelfasern liegen dichter und sie wirken visuell dicker.

Die Methoden mit Methionin-freiem bzw. mit Methionin-reduziertem Medium ergeben signifikant bessere Ergebnisse als die Standardmethode (Reduktion von FCS) oder die chemische Methode und dies bereits in normaler 2D-Kultur. Insbesondere ist die Fusionsrate höher (siehe auch: mehr Zellkerne in Myotuben), die Myotuben sind größer und nehmen eine größere Gesamtfläche ein. Tabelle 2 zeigt entsprechende Messergebnisse am Tag 4.

**Tabelle 2: Quantifizierung der Differenzierungsergebnisse (Tag 4)**

| | FCS | HS+Aza | -met +Glu | 2,5µm met |
|---|---|---|---|---|
| Zellkerne* gesamt | 2020±148^{A} | 2991±246^{B} | 2856±148 ^{B} | 2870±95 ^{B} |
| Zellkerne in Myotuben** | 90±14 ^{A} | 136±5 ^{A,B} | 466±72 ^{C} | 427±55 ^{C} |
| Anteil von Zellkernen in Myotuben (%) = Fusionsrate | 4±0,5 ^{A} | 5±0,4 ^{A,B} | 16±3 ^{C} | 15±2 ^{C} |
| Fläche an Myotuben (µm²) | 20±2 ^{A} | 16±1 ^{A,B} | 29±2 ^{C} | 34±3 ^{C} |
| Anzahl Myotuben | 75±4 | 74±6 | 57±9*** | 86±7 |
| Myotuben Gesamtfläche (%) | 12±1 ^{A} | 9±1 ^{A,B} | 18±1 ^{C} | 21±2^{C} |
| Ø Myotubengröße(µm²) | 0,3±0,03 ^{A} | 0,2±0,006 ^{A,B} | 0,5±0,07^{C,D} | 0,4±0,005^{A, C, D} |

Verschiedene Großbuchstaben (A-D) markieren signifikante (P<0,05) Unterschiede zwischen den jeweiligen Differenzierungsbedingungen. *Hier: Myonuclei; **Myotuben besitzen mindestens 2 Zellkerne / Myonuclei; ***Der niedrigere Wert resultiert aus einer Ablösung gebildeter Myotuben infolge einer zu hohen Myotubendichte im Kulturgefäß, die im Zusammenhang mit dem schneller ablaufenden Differenzierungsprozeß in Methionin-freiem Medium steht.

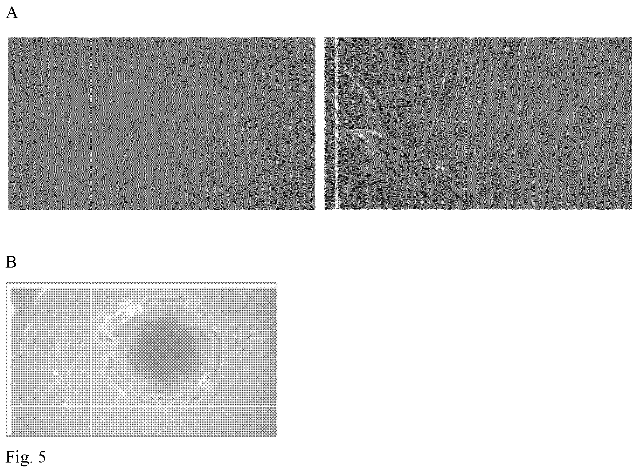

Differenzierung konnte auch mit UC-MSC in Methionin-freiem Medium erreicht werden (Fig. 5A). Ausserdem konnte gezeigt werden, dass UC-MSC in Methionin-freiem Medium bereits unter den Bedingungen einer konventionellen 2D-Kultur spontan Mikrogewebe bilden (Fig. 5B).

### Beispiel 7: Kultur in "hängenden Tropfen"

Um den Differenzierungsprozess weiter zu unterstützen, dem späteren Produkt Struktur bzw. Tiefe zu geben und eine fleischähnliche Textur zu erreichen, wurde das neue Differenzierungsmedium in Kombination mit der Kulturtechnik des "hängenden Tropfens" genutzt, um komplexe, dreidimensionale Mikrogewebe zu erzeugen. Diese Mikrogewebe können die Grundlage für die Herstellung Zell-basierter Fleischprodukte darstellen. Dies können neben geformtem Fleisch (Steak- oder Schnitzel-ähnlich), auch nicht geformte Fleischprodukte (z.B., ähnlich Gehacktem, Burger-Fleisch) oder Wurst sein.

Zellen wurden dazu in Differenzierungsmedium suspendiert und danach auf Deckel von Kulturgefäßen pipettiert die mit phosphatgepufferter Salzlösung gefüllt sind. Im Anschluss werden die Deckel auf die Gefäße gesetzt, um die Austrocknung der Kulturen zu verhindern. Getestet wurde das Verfahren wieder mit C2C12-Zellen in 2 Zelldichten und mit verschiedenen Differenzierungsmedien.

### Differenzierungsprotokoll: Schwerkraft

1. Herstellung einer Suspensionskultur
   - C2C12-Zellen (80% konfluent) 2x mit PBS waschen
   - Zellen kurz mit Trypsin-EDTA-Lösung (0,25% Trypsin, 0,53 mM EDTA) spülen, um Medium/FCS-Reste zu entfernen
   - 2 - 3 ml der Trypsin-EDTA-Lösung (0,25% Trypsin, 0,53 mM EDTA) in die Schale (10 cm) geben und unter dem Mikroskop beobachten, wann die Zellen abgelöst sind (etwa 5 - 15 min bei Raumtemperatur).
   - Lösen sich die Zellen schwer: bei 37°C inkubieren.
   - Frisches, komplettes (mit FCS) Wachstumsmedium (6 - 8 ml) zugeben, Zellen vorsichtig resuspendieren
   - Zellen in 15 ml-Röhrchen überführen
   - 40 µl einer 10 mg/ml DNAse-Stammlösung zugeben und 5 min bei RT inkubieren
   - Kurz vortexen und dann abzentrifugieren (200 x G, 5 min)
   - Überstand abnehmen und verwerfen, Zellen 2x mit 1 ml Wachstumsmedium waschen
   - Zentrifugieren und in 2 ml Medium aufnehmen, zählen und jeweils die Hälfte auf
      (1) 5 x 10⁵ Zellen/ml oder
      (2) 2.5 x 10⁶ Zellen/ml einstellen.
2. Hanging drop-Kultur etablieren
   - Boden einer 6 cm Kulturschale mit 5 ml PBS füllen
   - Den Deckel der Kulturschale umdrehen und mit einer Pipette 20 µl Tropfen (1; 10000 Zellen/Tropfen) bzw. 10 µl Tropfen (2; 25000 Zellen/Tropfen) in den Deckel pipettieren. z.B. bei Zelldichte (1) 10 x 20 µl Tropfen / Schale, mit Zelldichte (2) 20 x 10 µl Tropfen / Schale
   - Den Deckel auf die PBS-gefüllten Schalen setzen und in den Inkubator geben.
   - Inkubieren, bis sich Zellschichten bzw. Aggregate bilden, meist nach etwa 24 - 72h

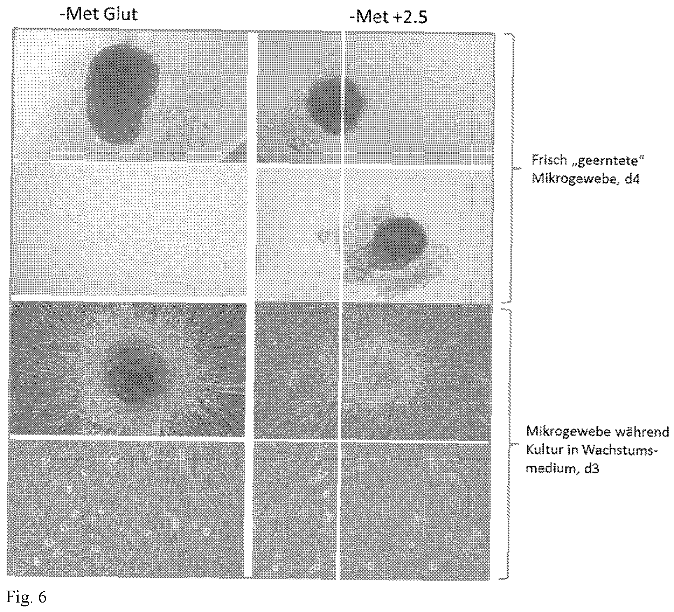

Die Bildung differenzierender, 3-dimensionaler Mikroaggregate erfolgte innerhalb von 2 - 3 Tagen, wobei ausschließlich die Zusammenlagerung und Interaktion der Zellen (Selbstorganisation) infolge der Schwerkraft und im Falle unseres neuen Differenzierungsmediums die natürlich ausgelöste Demethylierung den Prozess antreiben. Da die Zellen zur Bildung eigener extrazellulärer Matrixstrukturen angeregt wurden, mussten keine künstlichen Gerüstsubstanzen genutzt werden. Ergebnisbeispiele sind in Fig.6 gezeigt.

### Literatur:

AOAC International Official Methods for Analysis 17th Edition, AOAC International, Gaithersburg, MD, 2000
Arutyunyan et al., 2016. Stem Cells Internat Vol. 2016, dx.doi.org/10.1155/2016/6901286
Bastian et al. (1985), J Assoc Off Anal Chem 68(5):876-880
Beeravolu et al.(2016), Stem Cell Res 16: 696-711
Cardoso et al. (2012), BMC Biotechnology 12:18; www.biomedcentral.com/1472-6750/12/18
Carlin et al., 2006. Reprod Biol Endocrinol 4: 8; DOI: 10.1186/1477-7827-4-8
Conconi et al., 2006. Int J Mol Med 18:1089-1096
Corotchi et al., 2013. Stem Cell Res Ther 4:81; DOI: 10.1186/scrt232
Hoynowski et al., 2007. Biochem Biophysic Res Comm 362:347-353;
DOI:10.1016/j.bbrc.2007.07.182
Ishige et al., 2009. Int J Hematol 90:261-269
Kocaefe et al., 2010. Stem Cell Rev & Rep 6:512-522
Marcus-Sekura et al., 2011. Biologicals 39:359-369.
Moretti et al. 2010, AdvBiochem Engin/Biotechnol 123: 29-54
Pham et al., (2016). Cell Tissue Bank 17: 289-302
Shivakumar et al., 2016. J Cell Biochem 117: 2397-2412.
Simonne et al., (1997), Journal of the Science of Food and Agriculture 73(1):39-45
Stephens et al. 2018. Trends in Food Science & Technology 78: 155-166.
Thorrez & Vandenburgh. 2019. Nat Biotechnol 37: 215-226.
Wang et al., 2004. Stem Cells 22:1330-1337
Mrunalini K. Gaydhane, Urbashi Mahanta, Chandra S. Sharma, Mudrika Khandelwal, Seeram
Ramakrishna, Biomanufacturing Reviews, Bd. 3, Nr. 1, 19. März 2018
WO 2006/041429 A1

**Tabelle 1: Primer zum Nachweis von Genexpression, alle Primer sind von Sus scrofa-Sequenzen abgeleitet.**

| Gen | Sequenz (SEQ ID NO:) | Produktlänge | Referenz-Sequenz | Kommentar |
|---|---|---|---|---|
| NT5E (CD73) | fwd:CGTGGCGCGACTTTCTACCA (1) | 167 | XM_001927095.4 | - rev-Primer am Übergang Exon 2-3 |
| | rev: CCAGGGCCATGGCATCGTAA (2) | | | |
| THY-1 (CD90) | fwd: TCGCTCTCTTGCTAACAGTCTTGC (3) | 128 | NM_001146129.1 | - fwd-Primer am Übergang Exon 1-2 |
| | rev: CTGAATGGGCAGGTTGGTGGT (4) | | | |
| ENG (CD105) | fwd: TCAGCAACGAGGTGGTCGTC (5) | 243 | NM_214031.1 | - fwd-Primer am Übergang Exon 9-10, rev-Primer in Exon 12 |
| | rev: CCACGTCAGGCCCCAGATTC (6) | | | |
| Nanog | fwd: TCGACACCGAGACTGTCTCTCC (7) | 188 | ENSSSCT 00000062427.1 | - fwd-Primer am Übergang Exon 1-2 |
| | rev: ACAGAGCTGGGTCTGCGAGA (8) | | | |
| POU5F1 (Oct-4) | fwd: CCCGCCCTATGACTTCTGCG (9) | 220 | NM_001113060.1 | - rev-Primer am Exon- Übergang |
| | rev: CTGGGACTCCTCGGGGTTCG (10) | | | |
| Sox2 | fwd: CAGTGGTCAAGTCCGAGGCG (11) | 209 | NM_001123197.1 | - nur ein Exon known, keine Exon-überspannenden Primer |
| | rev: TGTACCGTTGATGGCCGTGC (12) | | | |
| CD14 | fwd: TGCCAAATAGACGACGAAGA (13) | 385 | NM_001097445.2 | - no exon spanning primer available since last 3 bases of exon one are CDS' start codon |
| | rev: ACGACACATTACGGAGTCTGA (14) | | | |
| CD34 | fwd: TGAAACCTCACTGCCTGCTGC (15) | 272 | NM_214086.1 | - fwd primer is exon spanning, primer pair also covers two introns |
| | rev: AGGGTCTTCGCCCAGCCTTTCT (16) | | | |

## Patentansprüche

1. Methode zur Erzeugung einer Zusammensetzung, welche tierisches Protein enthält, umfassend
(a) Isolierung von mesenchymalen Stammzellen aus einer Nabelschnur eines landwirtschaftlichen Nutztiers, bevorzugt aus der Familie Suidae, mehr bevorzugt aus der Gattung Sus, am meisten bevorzugt aus der Art Sus scrofa;
(b) Inkubieren der mesenchymalen Stammzellen unter Bedingungen, die zu einer myogenen Differenzierung der mesenchymalen Stammzellenführen, wobei die Bedingungen, die zu einer myogenen Differenzierung führen, Inkubation in einem Nährmedium umfassen, welches einen im Vergleich zum Standardmedium reduzierten Gehalt an Methionin von höchstens 5 µM aufweist; und
(c) Ernten der myogen differenzierten Zellen.

2. Methode nach Anspruch 1, wobei es sich bei den mesenchymalen Stammzellen um perinatale mesenchymale Stammzellen handelt.

3. Methode nach Anspruch 1 oder 2, wobei das Vermehren der mesenchymalen Stammzellen ein Einbringen der mesenchymalen Stammzellen in ein serumfreies Nährmedium umfasst, welches bevorzugt einen Anteil konditioniertes serumfreies Medium enthält.

4. Methode nach einem der Ansprüche 1 bis 3, wobei die Bedingungen, die zu einer myogenen Differenzierung führen, Inkubation in einem Nährmedium mit einer Konzentration an Methionin von höchstens 4 µM, bevorzugt höchstens 3 µM, noch mehr bevorzugt höchstens 2,5 µM und am meisten bevorzugt Inkubation in einem Nährmedium ohne Methionin (0 µM Methionin) umfassen.

5. Methode nach einem der Ansprüche 1 bis 4, wobei während der myogenen Differenzierung ein Kontakt der mesenchymalen Stammzellen mit einer festen Oberfläche vermieden wird, wodurch bevorzugt Mikrogewebe von myogen differenzierten mesenchymalen Stammzellen erzeugt werden.

6. Methode gemäß Anspruch 1, wobei Schritt (a) die folgenden Schritte umfasst:
(A) Bereitstellen von mindestens einem perinatalen Gewebe einer Nabelschnur eines landwirtschaftlichen Nutztiers;
(B) zerkleinern des perinatalen Gewebes, so dass Stücke davon erhalten werden,
(C) Inkubieren der Stücke des perinatalen Gewebes in einem Nährmedium;
(D) wodurch mesenchymale Stammzellen erhalten werden.

7. Methode nach Anspruch 6, wobei das Nährmedium in Schritt C) ein Nährmedium ist, welches frei von Serum eines landwirtschaftlichen Nutztieres ist, bevorzugt ein serumfreies Medium ist.

8. Verwendung eines Nährmediums, welches einen im Vergleich zum Standardmedium reduzierten Gehalt an Methionin von höchstens 5 µM aufweist, zur Differenzierung von mesenchymalen Stammzellen aus einer Nabelschnur eines landwirtschaftlichen Nutztiers.

9. Methode zur Herstellung einer fleischähnlichen Zusammensetzung, umfassend
I) myogene Differenzierung von mesenchymalen Stammzellen gemäß der erfindungsgemäßen Methode zur myogenen Differenzierung von mesenchymalen Stammzellen gemäß einem der Ansprüche 1 bis 7;
II) dreidimensionale Anordnung der in Schritt I) erhaltenen differenzierten mesenchymalen Stammzellen, und
III) Vermehren der Zellen in der in Schritt II) erhaltenen dreidimensionalen Anordnung.

10. Methode nach Anspruch 9, wobei die dreidimensionale Anordnung in Schritt II) durch Bioprinting von differenzierten mesenchymalen Stammzellen erhalten wird, bevorzugt durch Bioprinting von Mikrogeweben enthaltend differenzierte mesenchymale Stammzellen.

## Claims

1. Method for producing a composition which contains animal protein, comprising
(a) isolating mesenchymal stem cells from an umbilical cord of an agricultural animal, preferably from the family Suidae, more preferably from the genus Sus, most preferably from the species Sus scrofa;
(b) incubating the mesenchymal stem cells under conditions which lead to a myogenic differentiation of the mesenchymal stem cells, wherein the conditions which lead to a myogenic differentiation comprise incubation in a nutrient medium, which in comparison to the standard medium has a reduced content of methionine of at most 5 µM; and
(c) harvesting the myogenically differentiated cells.

2. Method according to Claim 1, wherein the mesenchymal stem cells are perinatal mesenchymal stem cells.

3. Method according to Claim 1 or 2, wherein the growth of the mesenchymal stem cells comprises introduction of the mesenchymal stem cells into a serum-free nutrient medium, which preferably contains a proportion of conditioned serum-free medium.

4. Method according to one of Claims 1 to 3, wherein the conditions which lead to a myogenic differentiation comprise incubation in a nutrient medium with a concentration of methionine of at most 4 µM, preferably at most 3 µM, still more preferably at most 2.5 µM, and most preferably incubation in a nutrient medium without added methionine (0 µM methionine).

5. Method according to one of Claims 1 to 4, wherein during the myogenic differentiation contact of the mesenchymal stem cells with a solid surface is avoided, whereby microtissues of myogenically differentiated mesenchymal stem cells are preferentially produced.

6. Method according to Claim 1, wherein step (a) comprises the following steps:
(A) providing at least one perinatal tissue of an umbilical cord of an agricultural animal;
(B) chopping the perinatal tissue, so that pieces thereof are obtained,
(C) incubating the pieces of the perinatal tissue in a nutrient medium;
(D) whereby mesenchymal stem cells are obtained.

7. Method according to Claim 6, wherein the nutrient medium in step (C) is a nutrient medium which is free from serum of an agricultural animal, and is preferably a serum-free medium.

8. Use of a nutrient medium which in comparison to the standard medium has a reduced content of methionine of at most 5 µM for the differentiation of mesenchymal stem cells from an umbilical cord of an agricultural animal.

9. Method for producing a meat-like composition, comprising
I) myogenic differentiation of mesenchymal stem cells according to the method of the invention for the myogenic differentiation of mesenchymal stem cells according to one of Claims 1 to 7;
II) three-dimensional structuring of the differentiated mesenchymal stem cells obtained in step I), and
III) growth of the cells in the three-dimensional structure obtained in step II).

10. Method according to Claim 9, wherein the three-dimensional structure in step II) is obtained by bioprinting of differentiated mesenchymal stem cells, preferably by bioprinting of microtissues containing differentiated mesenchymal stem cells.

## Revendications

1. Procédé de production d'une composition qui contient une protéine animale, comprenant
(a) l'isolement de cellules souches mésenchymateuses provenant d'un cordon ombilical d'un animal d'élevage agricole, de préférence de la famille des porcins, plus préférablement de l'espèce Sus, le plus préférablement de la sous-espèce Sus scrofa ;
(b) l'incubation des cellules souches mésenchymateuses dans des conditions qui conduisent à une différenciation myogène des cellules souches mésenchymateuses, les conditions qui conduisent à une différenciation myogène comprenant l'incubation dans un milieu nutritif qui présente, par rapport à un milieu standard, une teneur réduite en méthionine d'au plus 5 µM ; et
(c) la collecte des cellules différenciées de manière myogène.

2. Procédé selon la revendication 1, les cellules souches mésenchymateuses étant des cellules souches mésenchymateuses périnatales.

3. Procédé selon la revendication 1 ou 2, la multiplication des cellules souches mésenchymateuses comprenant une introduction des cellules souches mésenchymateuses dans un milieu nutritif exempt de sérum, qui contient de préférence une proportion de milieu exempt de sérum conditionné.

4. Procédé selon l'une des revendications 1 à 3, les conditions qui conduisent à une différenciation myogène comprenant une incubation dans un milieu nutritif présentant une concentration en méthionine d'au plus 4 µM, de préférence d'au plus 3 µM, encore plus préférablement d'au plus 2,5 µM et le plus préférablement une incubation dans un milieu nutritif sans méthionine (0 µM en méthionine).

5. Procédé selon l'une des revendications 1 à 4, un contact des cellules souches mésenchymateuses avec une surface solide étant évité pendant la différenciation myogène, suite à quoi on obtient de préférence des microtissus de cellules souches mésenchymateuses différenciées de manière myogène.

6. Procédé selon la revendication 1, l'étape (a) comprenant les étapes suivantes :
(A) mise à disposition d'au moins un tissu périnatal d'un cordon ombilical d'un animal d'élevage agricole ;
(B) fragmentation du tissu périnatal de manière à obtenir des morceaux de celui-ci,
(C) incubation des morceaux du tissu périnatal dans un milieu nutritif ;
(D) suite à quoi on obtient des cellules souches mésenchymateuses.

7. Procédé selon la revendication 6, le milieu nutritif dans l'étape C) étant un milieu nutritif qui est exempt de sérum d'un animal d'élevage agricole, de préférence un milieu exempt de sérum.

8. Utilisation d'un milieu nutritif, qui présente, par rapport à un milieu standard, une teneur réduite en méthionine d'au plus 5 µM, pour la différenciation de cellules souches mésenchymateuses provenant d'un cordon ombilical d'un animal d'élevage agricole.

9. Procédé pour la préparation d'une composition analogue à la viande, comprenant
I) la différenciation myogène de cellules souches mésenchymateuses selon le procédé selon l'invention pour la différenciation myogène de cellules souches mésenchymateuses selon l'une des revendications 1 à 7 ;
II) l'agencement tridimensionnel des cellules souches mésenchymateuses différenciées obtenues dans l'étape I) et
III) la multiplication des cellules dans l'agencement tridimensionnel obtenu dans l'étape II).

10. Procédé selon la revendication 9, l'agencement tridimensionnel dans l'étape II) étant obtenu par bio-impression de cellules souches mésenchymateuses différenciées, de préférence par bio-impression de microtissus contenant des cellules souches mésenchymateuses.
